# EUROPEAN PATENT APPLICATION

(11) **EP 1 747 785 A1**
(43) Date of publication of application: **31.01.2007**
(21) Application number: 05425560.9
(22) Date of filing: 28.07.2005
(51) Int. Cl.: A61K 31/724, A61P 7/00

(54) **Cyclodextrins for blood detoxification**

(71) Applicant: Humanitas Mirasole S.p.A., 20089 Rozzano (Milano) (IT); Istituto di Richerche Chimiche e Biochimiche G. Ronzoni, 20133 Milano (IT)
(72) Inventor: Graziani, Giorgio, 20125 Milano (IT); Naggi, Annamaria, 20025 Legnano Milano (IT); Torri, Giangiacomo, 20133 Milano (IT)
(74) Representative: Siniscalco, Fabio

(57) **Abstract**

The present invention relates to the use of cyclodextrins for the preparation of a medicament for the prevention of septic syndrome and a method for removing bacterial endotoxins (lipopolysaccharides) from blood.

## Description

. The present invention relates to a product for the prevention of septic syndrome and a method for the prevention of such disease.

. Septic syndrome (sepsis) is a serious complication of infection with gram negative bacteria.

**.** Frequently, said syndrome affects all organs and systems, resulting in multiple organ failure requiring the use of artificial devices (automatic respirators, artificial kidneys and cardiac stimulators). One particularly dreadful and widespread complication of sepsis is septic shock, which can even prove fatal.

**.** Sepsis is triggered by the liberation of exo- and endotoxins from the bacterial outer capsule. Said substances are lipopolysaccharides which, upon entering the circulatory system, trigger a series of reactions involving the humoral and cellular components of the immune system. The situations resulting in the greatest risk of this ailment are: serious trauma, perforations of the abdominal viscera, major abdominal surgery, especially in older diabetic or immune suppressed patients.

. Frequently, the body's own reaction to endotoxinaemia can provoke a series of responses involving the organ microcirculatory system (septic cascade).

. This reaction, despite being considered one of the body's defence mechanisms against bacterial invasion, is frequently abnormal, inducing the production of numerous mediators of inflammation (cytokines and other microproteins), responsible for the response's autoamplification process, causing serious damage to organs and systems.

. Treatment with antibiotics and substitution of the damaged organs with artificial devices does not always succeed in stemming the septic cascade.

. Hence, the most effective treatment against sepsis is the prevention of the body becoming invaded by bacterial toxins, before the latter can induce the septic cascade.

. Over the years, numerous blood detoxification techniques have been developed, however having the disadvantage of being not very efficient and entailing very high costs, largely due to the materials used.

. The aforementioned drawbacks are resolved by the use of cyclodextrins for the preparation of a medicament for the prevention of septic syndrome, and a method for the prevention of said disease as defined in the enclosed claims.

. In a first aspect, the present invention concerns the use of cyclodextrins for the preparation of a medicament for the prevention of septic syndrome.

**.** Cyclodextrins (CDs), also known as cycloamylose, cycloglucans, cyclomaltosides, are cyclic oligosaccharides constituted by the joining together, through α(1,4) bonds, of glucose units in numbers varying between 6 and 12. The term CD is preceded by a Greek letter indicating the number of glucose units present in the ring (α corresponds to 6 units, β corresponds to 7 units, etc.).

. Cyclodextrins are the enzymatic breakdown product of starch, by the enzyme glycosyl transferase (CGTase) produced by various bacteria (*e.g*.: *Bacillus Macerans, Klebsiella Pneumonite, Bacillus Stearothermophilus, Bacillus Megaterium* etc.).

. The enzyme CGTase cleaves the helical structure of the starch, while at the same time forming α(1,4) bonds between the glucose molecules, leading to the formation of cyclic oligoglucosides.

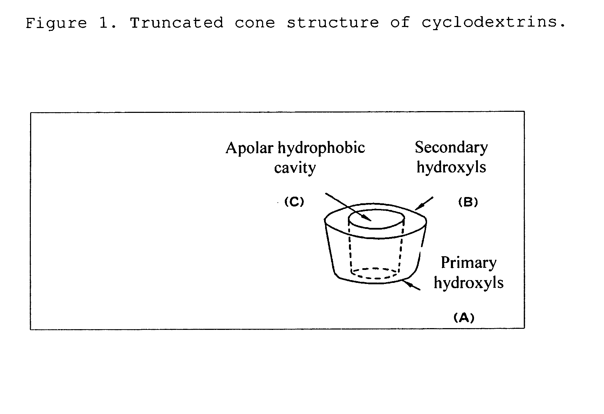

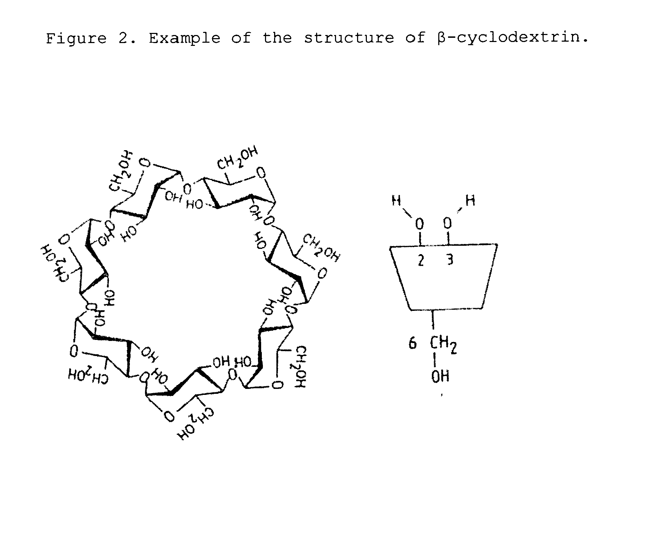

. Cyclodextrins, as shown in figures 1 and 2, have a truncated conical structure with the hydroxyl groups facing outwards, and the carbon and hydrogen atoms and the glycosidic bonds towards the inside of the structure. Furthermore, they have the primary hydroxyl groups positioned in the truncated cone area with the smaller diameter, and the secondary groups in the area with the larger diameter.

. This structure confers the CDs with particular properties: the high electron density, caused by the glycosidic oxygens, makes the cavity of the molecule apolar and hydrophobic and confers the exterior with a hydrophilic nature, thus cyclodextrins are soluble in water.

. Bacterial endotoxins or lipopolysaccharides (LPS) are characteristic components of the cell membrane of gram negative bacteria; they are not present in gram-positive bacteria.

**.** They are molecules constituted by a hydrophobic lipid chain which is responsible for the toxic properties, a central hydrophilic polysaccharide chain and a hydrophilic O-polysaccharide side chain, which is specific for each bacterial strain.

. Lipopolysaccharides have a tendency to form aggregates of various sizes, particularly micelles having molecular weights of approx. 1,000 KDa, in any solvent. Consequently, they may not be separated from blood by ultrafiltration, as they do not pass through the membrane pores and are hence retained along with the blood plasma components.

**.** The principle forming the basis of the invention is hence that of disrupting said large micelles thus allowing the lipopolysaccharides to be removed by ultrafiltration.

. For this purpose, cyclodextrins are used which are dissolved in an appropriate quantity of blood, or in plasma which has been previously separated from the remaining blood components.

**.** Once the cyclodextrins have been dissolved in blood or in plasma, they form a reversible but stable complex with the lipopolysaccharides, known as an inclusion complex.

. In particular, the hydrophobic cavity inside the cyclodextrins retains the bacterial endotoxins thus allowing the detoxification of the blood, which is then returned to clean circulation.

. In the majority of cases, the ratio between CDs and the bound molecule is one to one, and no covalent bonds are involved, however an associative-dissociative type equilibrium is established, mainly due to Van der Waals interactions and hydrogen bonding between the included molecule and the hydroxyl groups on the CD. For the purposes of the invention, the preferred cyclodextrins are: alpha- and gamma-cyclodextrin and the hydroxypropyl sulphate and ethanesulphonate derivatives of beta-cyclodextrin.

. Hence, in a second aspect, the present invention relates to a method for blood detoxification comprising the following steps:
a) Removing blood from a patient at risk of sepsis;
b) Preparing a suitable quantity of a cyclodextrin solution, possibly also comprising other components;
c) Adding the solution of step b) to the blood, allowing the formation of the cyclodextrin/lipopolysaccharide complex;
d) Ultrafiltration of the blood using a dialysis membrane: the blood components are retained by the membrane, while the cyclodextrin/lipopolysaccharide complex passes through the membrane and is eliminated. "High flux" dialysing membranes, allowing molecules with molecular weights within the range 40-50 KDa to pass, for example highly permeable polysulphone, may be used for the ultrafiltration of the blood and the separation of the cyclodextrin-polysaccharide complex;
e) Alternatively, prior to step c) an additional step b1), involving the separation of the plasma from the remainder of the blood, may be performed by inserting a plasma filter (polysulphone or a derivative thereof, for example polyethersulphone) on the arterial line of the extracorporeal circuit. Following separation, the blood plasma will be used in the subsequent steps c-d).
f) Combine the blood fraction retained by the filter with the previously separated fraction.

. Once steps e) or f) have been completed, the blood, thus detoxified, may be immediately retransferred to the patient.

. Advantageously, step d) is repeated several times, possibly using membranes with different pore sizes. The cyclodextrin solution in step b) is a physiological solution of cyclodextrin in concentrations ranging from 1 nM to 2000 nM, preferably from 1 nM to 200 nM and more preferably from 4 to 20 nM. Such values may vary depending on the presence of other molecules which may potentially compete with the lipopolysaccharides for complexing, and depending on the volume and number of vials which are administered to the patient. For example, the concentrations reported above preferably refer to 1 litre vials, while the concentrations would be increased if 100 ml vials were being used or would be decreased if several vials were being used.

. By physiological solution is meant an aqueous 0.9% solution of NaCl which is isotonic with blood.

. ADVANTAGES

**.** By using cyclodextrin dissolved in blood plasma, it is possible to eliminate bacterially released endotoxins by means of the simple and conventional ultrafiltration of the plasma. Hence, normal dialysis membranes may be used to achieve detoxification of the blood. All this leads to a substantial reduction in treatment costs, also bearing in mind that cyclodextrins are very inexpensive compounds. Another advantage of this method is the possibility for removing the pro and anti-inflammatory acting cytokines, considered to be important mediators of the immune response towards endotoxins and responsible for organ damage in sepsis, from the plasma of those patients. Such molecules are microproteins having molecular weights of approx. 15-30 KDa, whereby they may be removed by ultrafiltration of the plasma of septic patients.

## Claims

1. Use of cyclodextrins or derivatives thereof for the preparation of a medicament for the prevention of septic syndrome.

2. The use according to claim 1 wherein said cyclodextrins are selected from: alpha-cyclodextrin, gamma-cyclodextrin, beta-cyclodextrin sulphate, hydroxypropyl-beta-cyclodextrin ethanesulphonate-beta-cyclodextrin.

3. The use according to claims 1 or 2 for the removal of bacterial endotoxins or exotoxins from the blood.

4. The use according to any of the claims 1 to 2 wherein said endotoxins and exotoxins are lipopolysaccharides from gram-negative bacteria.

5. A method for the detoxification of blood comprising the following steps:
a) Removing blood from a patient at risk of sepsis;
b) Preparing a suitable quantity of a cyclodextrin solution, possibly also comprising other components;
c) Adding the solution of step b) to the blood, allowing the formation of the cyclodextrin/lipopolysaccharide complex;
d) Ultrafiltration of the blood using a dialysis membrane;
e) Combining the blood fraction retained by the filter with the previously separated fraction.

6. The method according to claim 5 comprising, following step f), a step involving re-transferring the purified blood to the patient.

7. The method according to claims 5 or 6 wherein step e) is repeated several times, possibly using membranes having different pore sizes.

8. The method according to any of the claims 5 to 7 wherein prior to step c) an additional step b1) is performed involving the separation of the plasma from the remaining blood fractions.

9. The method according to any of the claims 5 to 8 wherein the cyclodextrin solution of step c) is a physiological solution having a concentration ranging from 1 nM to 2000 nM, preferably from 1 nM to 200 nM and more preferably from 4 to 20 nM.
